# EUROPEAN PATENT SPECIFICATION

(11) **EP 1 383 718 B1**
(45) Date of publication and mention of the grant of the patent: **28.06.2006**
(21) Application number: 02722977.2
(22) Date of filing: 12.04.2002
(51) Int. Cl.: C07B 41/06, C07C 67/347, C07C 67/54, C07C 69/716, C07C 45/50

(54) **CONTINUOUS HYDROFORMYLATION PROCESS**
KONTINUIERLICHES HYDROFORMYLIERUNGSVERFAHREN
PROCEDE D'HYDROFORMYLATION CONTINU

(30) Priority: 13.04.2001 EP 01201371
(43) Date of publication of application: 28.01.2004
(73) Proprietor: DSM IP Assets B.V., 6411 TE Heerlen (NL)
(72) Inventor: TINGE, Johan, Thomas, NL-6137 LL Sittard (NL); SMITS, Hubertus, Adrianus, NL-6213 CA Maastricht (NL)
(74) Representative: Verhaegen, Ilse Maria M.
(86) International application number: PCT/NL2002/000240
(87) International publication number: WO 2002/083605

(56) References cited:
- EP-A- 0 839 787
- EP-A- 0 839 794
- US-A- 4 792 636

## Description

The invention relates to a continuous hydroformylation process for forming an aldehyde comprising:
A) reacting an olefinically unsaturated compound containing from 6 to 30 carbon atoms, carbon monoxide, hydrogen, and a rhodium-bisphosphite ligand complex catalyst to form a reaction mixture,
B) separating aldehyde product from at least a portion of said reaction mixture, resulting in an aldehyde containing product stream and a catalyst containing product stream, and
C) recycling the catalyst containing stream to A).

It is well known in the art that organophosphites may be employed as catalyst ligands for rhodium based hydroformylation catalysts and that such catalysts exhibit exceptional activity and regioselectivity for producing aldehydes via olefin hydroformylation. For instance, EP 839787, EP 839794, U.S. 4668651, 4769498 and US 4792636 fully detail such hydroformylation.

However, despite the benefits attendant with such rhodium-organophosphite complex catalyzed hydroformylation processes, stability of the ligand and catalyst remains a primary concern. Although hydroformylation processes are performed at moderate temperatures (<110°C), heating rhodium organophosphite catalysts above these temperatures has led to decomposition of the catalyst and ligand and has generally resulted in Irreversible ligand and/or rhodium metal loss. For example U.S. Pat. Nos 5,288,918 and 5,763,670 describe the decomposition of rhodium bisorganophosphite complexes and ligands which occurs as the result of elevated temperatures. As a result of this decomposition, the activity and efficiency of the hydroformylation process are lowered and the cost of operating a hydroformylation facility is greatly increased.

The thermal decomposition of rhodium bisorganophosphite complexes and/or of free bisorganophosphite ligand is most apparent when the desired aldehyde product is separated from the catalyst by means of evaporation, especially when aldehydes with high boiling points (aldehydes with at least 6 carbon atoms) are to be separated. When producing aldehydes with high boiling points (aldehydes with at least 6 carbon atoms), high temperatures are required to vaporize these compounds so they may be separated and isolated from other materials which are present in the reaction mixture. These harsh conditions inevitably result in rhodium complex catalyst decomposition and/or in free ligand decomposition during product isolation. A technique aimed at aldehyde separation at lower temperatures, is membrane separation as for example described in WO-A-9634687. However, the use of membrane separation has the disadvantage that it is difficult or even impossible to achieve an economic and effective separation of the desired aldehyde product from the catalyst. Another hydroformylation process with an improved hydroformylation procedure whereby exposure of the catalyst-containing medium to catalyst deactivating conditions is obviated and whereby the recovery of product aldehydes is effected at a maximum temperature which is close as possible to that prevailing in the hydroformylation zone is described in US 4792636. Other techniques to prevent or minimize degradation of polydentate phosphite ligands are based on a process performed in the presence of an additional compound. For example in EP 839787 a monodentate phosphine is added and in EP 839794 an acid compound having a pKa between 1 and 12 measured in water at 18 °C is added to prevent or minimize degradation of polydentate phosphite ligands.

The object of the invention is to provide an improved process for separating rhodium complex catalyst and aldehyde product(s) in a rhodium-organophosphite complex catalyzed hydroformylation process.

This object is achieved in that said separation is accomplished in a vacuum evaporator and a vapour/liquid separator and wherein the temperature of the heating surface of the evaporator is less than 125 °C and the residence time of the catalyst in the evaporator is less than 15 minutes.

It has been found that in the process of the present invention the desired aldehyde product can be effectively separated from the catalyst and in addition the rhodium complex catalyst decomposition during product isolation is avoided or at least minimized. With effective separation is meant that the aldehyde containing product stream does not substantially contain catalyst. Effective separation of the desired aldehyde product from the catalyst is advantageous because catalyst present in the aldehyde containing product stream may be lost during downstream processing steps. An additional advantage of the process of the present invention is that the separation can be performed in a very economic way.

In a preferred embodiment of the hydroformylation process of the present invention, the evaporation is performed at a pressure below 200 mbar, more preferably below 50 mbar, more preferably below 20 mbar, and still more preferably the pressure is below 10 mbar.

The mean residence time of the catalyst in the evaporater is preferably less than 10 minutes and more preferably less than 5 minutes. Preferably the heating surface of the evaporator of the present invention is maintained at a temperature below 125°C, and more preferably this surface is maintained below 110°C. The higher the temperature of the heating surface, the lower the residence time of the catalyst in the evaporator. The temperature of the heating surface and the residence time of the catalyst in the evaporator will depend upon the aldehyde to be separated.

Accordingly, the subject invention encompasses effective separating of the desired aldehyde product from the catalyst and encompasses preventing or minimizing the catalyst deactivation of rhodium-bisphosphite complex catalyzed hydroformylation processes for producing aldehydes, by carrying out the evaporation step of said process with a vacuum evaporator with a short mean residence time and moderate thermal exposure of the catalyst in combination with a vapor liquid separator. Suitable vacuum evaporators in which the evaporation of the process of the present invention can be performed are falling film evaporators, wiped film evaporators or short path evaporators. Such vacuum evaporators consist of a heating surface which heats at least a portion of the reaction mixture as a thin liquid film, resulting in a liquid mixture comprising the high boiling compounds and a vapour mixture comprising the low boiling compounds.The vapour mixture is usually condensed in a condensation functionality. The evaporation functionality and condensation functionality may be physically separated into separate housings as in a falling film evaporator and in a wiped film evaporator with an external condenser or it may be integrated into a single housing as in a short path evaporator. For the purposes of this invention, the term reaction mixture is defined as the liquid solution present within the reaction vessel.

It is also preferable to perform the evaporation of the present invention by distilling a thin film of the reaction mixture to separate the product aldehyde, and such a technique is a preferred embodiment of the present invention. Preferably the reaction mixture film of the present invention has a thickness between 0.05 - 5 mm. Still more preferably, the film thickness is between 0.05 and 0.5 mm. It is also advantageous to mechanically wipe the heating surface, thereby obtaining improved heat and mass transfer and preventing thermal and concentration gradients from being formed along the axis of the liquid film thickness. Wiping also makes the residence time distribution more narrow which is beneficial to the catalyst stability. It has however been found that although the mixture to be separated might become a rather viscous mixture during evaporation, the evaporation can advantageously be performed in a falling film evaporator. It was initially contemplated having the evaporation to proceed in a short-path evaporator or in a wiped film evaporator in order to minimize the catalyst decomposition and to maximize the amount of catalyst separated from the desired aldehyde. It has now been found that the use of a short path evaporator or a wiped film evaporator is suitable, but not a prerequisite, and that the evaporation can advantageously be performed in a falling film evaporator. In general, the falling film evaporator (FFE) is a cylindrical apparatus with a heat source on the wall (so called heating surface). The mixture to be separated is in general fed to the top of the FFE and flows down the heating surfaces as a film. The film is heated, typically by means of indirect heat exchange with a heating medium through the wall of the cylindrical vessel, for example oil or steam. Under the heating, the lower boiling components of the mixture to be separated are caused to evaporate. The lower boiling components are removed from the evaporator as a vapour and the higher boiling components are removed from the evaporator as a liquid. Preferably a part of the liquid is recycled to the vacuum evaporator and a part, optionally after treatment thereof, is recycled to the reactor. In the process of the invention, the vapour containing desired aldehyde product is subsequently fed to a vapour liquid separator. Suitable vapour liquid separators are cyclones or wires. The vapour removed from the vapour/liquid separator is preferably subsequently condensed in a separate condenser (so-called condensation functionality). The vapour liquid separator is advantageously placed between the evaporation functionality and the condensation functionality. A preferred vapour liquid separator used in the process of the present invention is a cyclone. Depending on the particle size of the liquid droplets in the vapour, a suitable cyclone can be chosen by a man skilled in the art.

In a preferred embodiment of the invention, the reaction mixture removed from the hydroformylation reactor is subjected to a pressure reduction so as to volatilize and remove a substantial portion of the unreacted gases dissolved in the reaction mixture and then pass the so-obtained liquid reaction mixture which now contains a much lower syn gas concentration than was present in the reaction mixture leaving the hydroformylation reaction zone to a separator, preferably an evaporator in which the compounds having a lower boiling point than the desired aldehyde product are separated from the reaction mixture. The liquid mixture leaving this evaporator is fed to the separation section in which the desired aldehyde product is substantially separated from the catalyst comprising the vacuum evaporator in combination with the vapour liquid separator.

In general, such hydroformylation reactions involve the production of aldehydes by reacting an olefinic compound with carbon monoxide and hydrogen in the presence of a rhodium-bisphosphite complex catalyst in a liquid medium that may also contains a solvent for the catalyst The process may be carried out In a continuous single pass mode or more preferably in a continuous liquid catalyst recycle manner. The recycle procedure generally involves withdrawing a portion of the liquid reaction medium containing the catalyst and aldehyde product from the hydroformylation reaction zone, either continuously or intermittently, and distilling the aldehyde product therefrom in one or more stages, in a separate distillation zone in order to recover the aldehyde product and other volatile materials in vaporous form, the non-volatilized rhodium catalyst containing residue being recycled to the reaction zone. Likewise, the recovered non-volatilized rhodium catalyst containing residue can be recycled with or without further treatment to the hydroformylation zone in any conventional manner desired. Accordingly, the processing techniques of this invention may correspond to any known processing techniques such as heretofore employed in conventional liquid catalyst recycle hydroformylation reactions.

As noted above the hydroformylation reaction conditions that may be employed in the hydroformylation processes encompassed by this invention may include any suitable continuous hydroformylation conditions heretofore disclosed in the above-mentioned patents. For instance, the total gas pressure of hydrogen, carbon monoxide and olefinic unsaturated starting compound of the hydroformylation process may range from about 0.007 MPa to about 69 MPa (about 1 to about 10,000 psia). In general, however, it is preferred that the process be operated at a total gas pressure of hydrogen, carbon monoxide and olefinic unsaturated starting compound of less than about 10.3 MPa (1500 psia) and more preferably less than about 3.4 MPa (500 psia). The minimum total pressure being limited predominately by the amount of reactants necessary to obtain a desired rate of reaction. More specifically the carbon monoxide partial pressure of the hydroformylation process of this invention is preferable from about 0.007 MPa to about 0.8 MPa (about 1 to about 120 psia), and more preferably from about 0.02 MPa to about 0.6 MPa (about 3 to about 90 psia), while the hydrogen partial pressure is preferably about 0.1 MPa to about 1.1 MPa (about 15 to about 160 psia) and more preferably from about 0.2 MPa to about 0.7 MPa (about 30 to about 100 psia). In general H₂:CO molar ratio of gaseous hydrogen to carbon monoxide may range from about 1:10 to 100:1 or higher, the more preferred hydrogen to carbon monoxide molar ratio being from about 1:10 to about 10:1. Further, the hydroformylation process may be conducted at a reaction temperature from about 45° C to about 150°C. In general hydroformylation reaction temperature of about 50°C to about 120°C are preferred for all types of olefinic starting materials, the more preferred reaction temperatures being from about 50° C to about 100° C and most preferably about 95° C.

The olefinic starting material reactants that may be employed in the hydroformylation reactions encompassed by this invention are olefinic compounds containing from 6 to 30 carbon atoms. Such olefinic compounds can be terminally or internally unsaturated and be of straight-chain, branched chain or cyclic structures, as well as be olefin mixtures, such as obtained from the oligomerization of propene, butene, isobutene, etc., (such as so called dimeric, trimeric or tetrameric propylene, and the like, as disclosed, e.g., in U.S. Pat. Nos. 4,518,809 and 4,528,403). Moreover, such olefinic compounds may further contain one or more ethylenic unsaturated groups, and of course, mixtures of two or more different olefinic compounds may be employed as the starting hydroformylation material if desired. Further such olefinic compounds and the corresponding aldehyde products derived therefrom may also contain one or more groups or substituents which do not unduly adversely affect the hydroformylation process or the process of this invention such as described, e.g., in U.S. Pat. Nos. 3,527,809 and 4,668,651.

Illustrative olefinic unsaturated compounds are alpha-olefins, internal olefins, alkyl alkenoates such as methyl-2-pentenoate, methyl-3-pentenoate and methyl-4-pentenoate, alkenyl alkanoates, alkenyl alkyl ethers, alkenols, and the like, e.g., 1-hexene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, 2-hexene, 3-hexane, 2-heptene, cyclohexene, propylene dimers, propylene trimers, propylene tetramers, 2-ethyl-1-hexene, 2-octene, styrene, 3-phenyl-1-propene, 1,4-hexadiene, 1,7-octadiene, 3-cyclohexyl-1-butene, , allyl butyrate, hex-1-en-4-ol, oct-1-en-4-ol, , 3-butenyl acetate, n-propyl-7-octenoate, 5-hexenamide, 4-methyl styrene, 4-isopropyl styrene, 4-tert-butyl styrene, alpha-methyl styrene, 4-tert-butyl-alpha-methyl styrene, 1,3-diisopropenylbenzene, eugenol, iso-eugenol, safrole, iso-safrole, anethol, 4-allylanisole indene, limonene, betapinene, dicyclopentadiene, cyclooctadiene, camphene, and linalool.

Of course, it is understood that mixtures of different olefinic starting materials can be employed, if desired, by the hydroformylation process of the subject invention. More preferably the subject invention is especially useful for the production of aldehydes, by hydroformylating alpha olefins containing from 6 to 20 carbon atoms, and internal olefins containing from 6 to 20 carbon atoms as well as starting material mixtures of such alpha olefins and internal olefins. Still more preferably the subject invention is especially useful for the production of alkyl formylvalerates from alkyl-pentenoates, in any isomeric form, or mixture thereof. These include alkyl-4-pentenonate, trans-alkyl-3-pentenonate, cis-alkyl-3-pentenonate, trans-alkyl-2-pentenonate and cis-alkyl-2-pentenonate. In a preferred embodiment of the present invention, the aldehyde produced is alkyl-5-formylvalerate. Alkyl-5-formylvalerate or a mixture of alkyl-5-, alkyl-4-, alkyl-3- and alkyl-2-formylvalerate is prepared by reaction of alkyl-3-pentenoate or a mixture of alkyl-4-, alkyl-3- and alkyl-2-pentenoate with carbon monoxide and hydrogen in the presence of a hydroformylation catalyst. Still more preferably the subject invention is especially useful for the production of aldehydes from methyl-3-pentenonate, in any isomeric form, or mixture of isomeric forms. It is also to be understood that commercial alpha olefins containing 6 or more carbon atoms may contain minor amounts of corresponding internal olefins and/or their corresponding saturated hydrocarbon and that such commercial olefins need not necessarily be purified from same prior to being hydroformylated.

The present invention therefore also relates to a continuous hydroformylation process for forming methyl-5-formylvalerate comprising:
A) reacting methyl-3-pentenoate, carbon monoxide, hydrogen, and a rhodium-bisphosphite ligand complex catalyst to form a reaction mixture comprising methyl-5-formylvalerate and catalyst,
B) separating methyl-5-formylvalerate from at least a portion of said reaction mixture, resulting in a methyl-5-formylvalerate containing product stream and a catalyst containing product stream, and
C) recycling the catalyst containing stream to A),
wherein said separation is accomplished in a vacuum evaporator in combination with a vapour/liquid separator and wherein the temperature of the heating surface of the evaporator is less than 125°C and the residence time of the catalyst in the evaporator is less than 15 minutes.

A preferred embodiment of the process of the invention is a continuous hydroformylation process for forming methyl-5-formylvalerate comprising:
A) reacting methyl-3-pentenoate, carbon monoxide, hydrogen, and a rhodium-bisphosphite ligand complex catalyst to form a reaction mixture comprising methyl-5-formylvalerate and catalyst,
B) separating methyl-5-formylvalerate from at least a portion of said reaction mixture, resulting in a methyl-5-formylvalerate containing product stream and a catalyst containing product stream, and
C) recycling the catalyst containing stream to A),
wherein said separation is accomplished in a vacuum falling film evaporator in combination with a vapour/liquid separator and preferably in combination with a cyclone.

In this preferred embodiment of the invention, the reaction mixture leaving the hydroformylation reaction zone comprises methyl-5-formylvalerate, by-products, any unconverted methyl-3-pentenoate, catalyst, carbon monoxide and hydrogen. In a more preferred embodiment of the invention, at least a part of this reaction mixture is subjected to a pressure reduction in a flasher to for example atmospheric pressure. The released carbon monoxide and hydrogen are preferably recycled to the hydroformylation reaction zone. Subsequently the mixture leaving the flasher is fed to a separating section (1) comprising a vacuum evaporator operated at a pressure of between 50 and 500 mbar, preferably at a pressure of between 50 and 200 mbar. The gaseous mixture leaving this separating section usually comprises methyl-2-pentenoate, methyl-4-pentenoate and unconverted methyl-3-pentenoate. The residual liquid mixture of the separating section (1) is preferably subsequently fed to separation section (2) comprising preferably a falling film evaporator in combination with a cyclone. The use of separating section (1) is advantageous as this results in an improved heat and mass transfer in the vacuum evaporator of separating section (2).

As noted above, the continuous hydroformylation process of this invention involved the use of a rhodium-bisphosphite ligand complex catalyst as described herein. Of course mixtures of such catalysts can also be employed if desired. The amount of rhodium-phosphite complex catalyst present in the reaction medium of a given hydroformylation process encompassed by this invention need only be that minimum amount necessary to provide the given rhodium concentration desired to be employed and which will furnish the basis for at least the catalytic amount of rhodium necessary to catalyze the particular hydroformylation process involved such as disclosed e.g. in the above-mentioned patents. In general, rhodium concentrations in the range of from about 10 ppm to about 1000 ppm, calculated as free rhodium, in the hydroformylation reaction medium should be sufficient for most processes, while it is generally preferred to employ from about 10 to 500 ppm of rhodium and more preferably from 25 to 350 ppm to rhodium.

In addition to the rhodium-bisphosphite ligand complex catalyst the hydroformylation process encompassed by this invention may be carried out in the presence of free bisphosphite ligand, i.e. ligand that is not complexed with the rhodium metal of the complex catalyst employed. Said free bisphosphite ligand may correspond to any of the above defined bisphosphite ligands discussed above as employable herein. When employed it is preferred that the free bisphosphite ligand be the same as the bisphosphite ligand of the rhodium-bisphosphite complex catalyst employed. However, such ligands need not be the same in any given process. Moreover, while it may not be absolutely necessary for the hydroformylation process to be carried out in the presence of any such free bisphosphite ligand, the presence of at least some amount of free bisphosphite ligand in the hydroformylation reaction medium is preferred, Thus the hydroformylation process of this invention may be carried out in the absence or presence of any amount of free bisphosphite ligand, e.g. up to 100 moles, or higher per mole of rhodium metal in the hydroformylation reaction medium. Preferably the hydroformylation process of this invention is carried out in the presence of from about 1 to about 50 moles of bisphosphite ligand, and more preferably from about 1 to about 4 moles of bisphosphite ligand, per mole of rhodium metal present in the reaction medium; said amounts of bisphosphite ligand being the sum of both the amount of bisphosphite ligand that is bound (complexed) to the rhodium metal present and the amount of free (non-complexed) bisphosphite ligand present. Of course, if desired, make-up or additional bisphosphite ligand can be supplied to the reaction medium of the hydroformylation process at any time and in any suitable manner, e.g. to maintain a predetermined level of free ligand in the reaction medium.

The hydroformylation reactions encompassed by this invention may also be conducted in the presence of an organic solvent for the rhodium-bisphosphite complex catalyst and any free bisphosphite ligand that might be present. Any suitable solvent which does not unduly adversely interfere with the intended hydroformylation process can be employed. Illustrative suitable solvents for rhodium catalyzed hydroformylation processes include those disclosed e.g. in U.S. Pat. No. 4,668,651. Of course mixtures of one or more different solvents may be employed if desired. Most preferably the solvent will be one in which the olefinic starting material, catalyst, and weakly acidic additive if employed, are all substantially soluble. In general, it is preferred to employ aldehyde compounds corresponding to the aldehyde products desired to be produced and/or higher boiling aldehyde liquid condensation by-products as the primary solvent, such as the higher boiling aldehyde liquid condensation by-products that are produced in situ during the hydroformylation process. Indeed, while one may employ any suitable solvent at the start up of a continuous process, the primary solvent will normally eventually comprise both aldehyde products and higher boiling aldehyde liquid condensation by-products due to the nature of such continuous processes. Such aldehyde condensation by-products can also be performed if desired and used accordingly. Of course, the amount of solvent employed is not critical to the subject invention and need only be that amount sufficient to provide the reaction medium with the particular rhodium concentration desired for a given process. In general, the amount of solvent may range from 0 percent by weight up to about 95 percent by weight or more based on the total weight of the reaction medium.

In a preferred embodiment of the present invention the rhodium-bisphosphite complex catalyst comprises a bisphosphite ligand of a formula selected from the group consisting of: 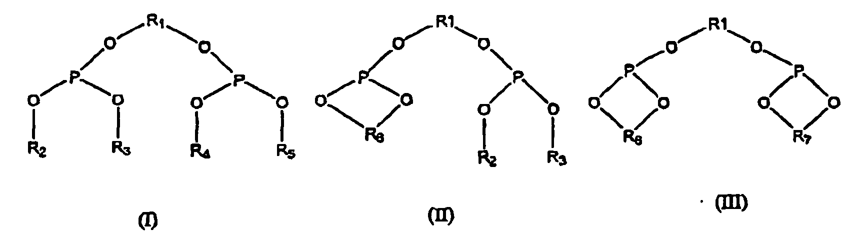 wherein each R¹ represents a divalent radical selected from a group consisting of alkylene, alkylene-(Q)ₙ-alkylene, arylene and arylene-(Q)ₙ-arylene, and wherein each alkylene radical individually contains from 2 to 18 carbon atoms and is the same or different, and wherein each arylene radical individually contains from 6 to 18 carbon atoms and is the same or different, wherein each Q individually represents a divalent bridging group of -O- or-CR'R"- wherein each R' and R" radical individually represents hydrogen or a methyl radical; and wherein each n individually has a value of 0 or 1,
wherein R², R³, R⁴, and R⁵ might be the same or different and each is individually represented by the structure of (VI) or (VII), 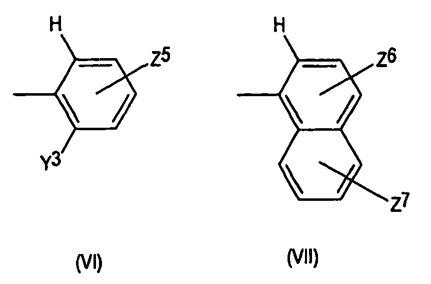 wherein R⁶ and R⁷ might be the same or different and each is individually represented by the structure of (VIII) or (IX), 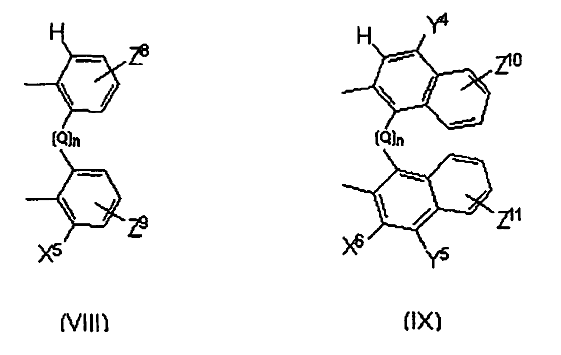 wherein X⁵ and X⁶ might be the same or different and each individually represents a hydrogen or an organic radical, wherein Y³, Y⁴ and Y⁵ are the same or different and each represents a hydrogen or alkyl radical, wherein Z⁵, Z⁶, Z⁷, Z⁸, Z⁹, Z¹⁰ and Z¹¹ might be the same or different and each represent a hydrogen or an organic radical placed at any remaining position of the aryl rings.

In a more preferred embodiment of the present invention, R¹ is represented by the structure of (IV),(V), 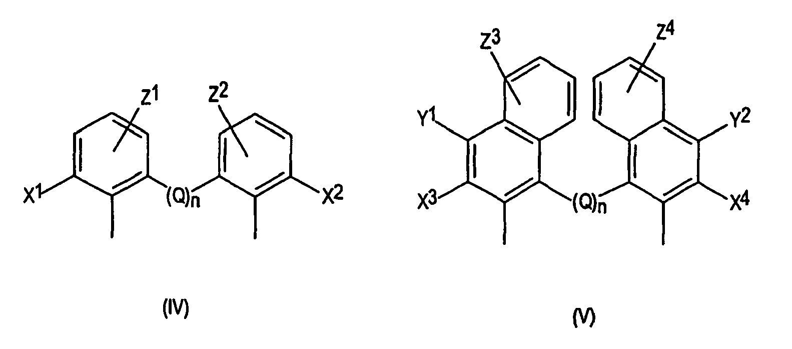 (VIII), (IX), wherein (Q)ₙ is the same as above, wherein X¹, X², X³, X⁴, X⁵ and X⁶ might be the same or different and each individually represents a hydrogen or an organic radical, wherein Y¹, Y², Y⁴ and Y⁵ are the same or different and each represents a hydrogen or alkyl radical, wherein Z¹, Z², Z³, Z⁴, Z⁸, Z⁹, Z¹⁰ and Z¹¹might be the same or different and each represent a hydrogen or an organic radical placed at any remaining position of the aryl rings.

In an even more preferred embodiment of the present invention R¹ is represented by the structure of (IV),(V), (VIII), (IX), wherein (Q)ₙ is the same as above, wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁸ is the same as Z⁹ in Formula (VIII), Z¹⁰ is the same as Z¹¹ and Y⁴ and Y⁵ are hydrogen radicals in Formula (IX).

Still more preferably said hydroformylation process is performed wherein said ligand used is chosen from the group consisting of [3,3'-bis(t-butyl)-5,5'-dimethoxy-1,1'-biphenyl-2,2'-diyl]-bis(oxy)]-bis(dibenzo[d,f] [1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.

Illustrative rhodium-bisphosphite complex catalyzed continuous hydroformylation processes in which such catalyst deactivation may occur include hydroformylation processes such as described, e.g., in U.S. Pat. Nos. 4,668,651; 4,774,361; 4,769,498; and 5,288,918 wherein the bisphosphite ligand is a ligand selected from the class consisting of Formulas (I)-(III) above, the entire disclosures of said patents being incorporated herein by reference thereto. Thus such hydroformylation processes and the conditions thereof are well known and it is to be understood that the particular manner in which the hydroformylation reaction is carried out and particular hydroformylation reaction conditions employed may be varied widely and tailored to meet individual needs and produce the particular aldehyde product desired.

Illustrative rhodium-bisphosphite complex catalysts employable in such hydroformylation reactions encompassed by this invention may include those disclosed in the above mentioned patents wherein the bisphosphite ligand is a ligand selected from the class consisting of Formulas (I), (II) and (III) above. In general, such catalysts may be preformed, or formed in situ, as described e.g., in said U.S. Pat. Nos. 4,668,651 and 4,769,498, and consist essentially of rhodium in complex combination with the organobisphosphite ligand. It is believed that carbon monoxide is also present and complexed with the rhodium in the active species. The active catalyst species may also contain hydrogen directly bonded to the rhodium.

As noted above illustrative organobisphosphite ligands that may be employed as the bisphosphite ligand complexed to the rhodium catalyst and/or any free bisphosphite ligand (i.e. ligand that is not complexed with the rhodium metal in the active complex catalyst) in such hydroformylation reactions encompassed by this invention include those of Formulas (I), (II), and (III) above.

Illustrative divalent radicals represented by R¹ in the above bisphosphite formulas (I), (II) and (III) include substituted and unsubstituted radicals selected from the group consisting of alkylene, alkylene-(Q)ₙ-alkylene, phenylene, naphthylene, phenylene-(Q)ₙ-phenylene and naphthylene-(Q)ₙ₋naphthylene radicals, and where Q, and n are the same as defined above. More specific illustrative divalent radicals represented by R¹ are shown by the structure of (IV) or (V) wherein (Q)ₙ is the same as above. These include, 1,1'biphenyl-2,2'-diyl, 3,3'-dialkyl-1,1'-biphenyl-2,2'-diyl, 3,3'-dicarboxy ester-1,1'-biphenyl-2,2'-diyl, 1,1'binaphthyl-2,2'-diyl, 3,3'-dicarboxy ester-1,1'-binaphthyl-2,2'-diyl, 3,3'-dialkyl-1,1'-binaphthyl-2,2'-diyl, 2,2'-binaphthyl-1,1'-diyl, phenylene-CH₂₋phenylene, phenylene-O-phenylene, phenylene-CH(CH₃)-phenylene radicals, and the like.

Illustrative radicals represented by Z¹, Z², Z³, Z⁴, Z⁵, Z⁶, Z⁷, Z⁸, Z⁹, Z¹⁰, and Z¹¹ in above Formulas (IV) to (IX), in addition to hydrogen, include any of those organic substituents containing from 1 to 18 carbon atoms, disclosed in U.S. Pat. No. 4,668,651, or any other radical that does not unduly adversely effect the process of this invention. Illustrative radicals and substituents encompass alkyl radicals, including primary, secondary and tertiary alkyl radicals such as methyl, ethyl n-propyl, isopropyl, butyl, sec-butyl, t-butyl, neo-pentyl, n-hexyl, amyl, sec-amyl, t-amyl, iso-octyl, decyl, octadecyl, and the like; aryl radicals such as phenyl, naphthyl and the like; aralkyl radicals such as benzyl, phenylethyl, triphenylmethyl, and the like; alkaryl radicals such as tolyl, xylyl, and the like; condensated aryl radicals such as phenylene, naphthylene, and the like, alicyclic radicals such as cyclopentyl, cyclohexyl, 1-methylcyclohexyl, cyclooctyl, cyclohexylethyl, and the like; alkoxy radicals such as methoxy, ethoxy, propoxy, t-butoxy-OCH₂CH₂OCH₃, -O(CH₂CH₂)₂OCH₃, -O(CH₂CH₂)₃OCH₃, and the like; aryloxy radicals such as phenoxy and the like; as well as silyl radicals such as -Si(CH₃)₃, -Si(OCH₃)₃, -Si(C₃H₇)₃, and the like; amino radicals such as -NH₂, -N(CH₃)₂, -NHCH₃, -NH(C₂H₅), and the like; acyl radicals such as -C(O)CH₃, -C(O)C₂H₅, -C(O)C₆H₅, and the like; carbonyloxy radicals such as -C(O)OCH₃, -C(O)OCH(CH₃)₂ -(C(O)CH(CH₃)C₈H₁₇, and the like; oxycarbonyl radicals such as -O(CO)C₆H₅, and the like; amido radicals such as -CONH₂, -CON(CHg)₂, -NHC(O)CH₃, and the like; sulfonyl radicals such as -S(O)₂C₂H₅ and the like; sulfinyl radicals such as -S(O)CH₃ and the like; thionyl radicals such as -SCH₃, -SC₂H₅, -SC₆H₅, and the like; phosphonyl radicals such as -P(O)(C₆H₅)₂, -P(O)(CH₃)₂, -P(O)(C₂H₅)₂, -P(O)(C₃H₇)₂, -P(O)CH₃(C₆H₅), -P(O)(H)(C₆H₅), and the like.

Illustrative radicals represented by X¹, X², X³, X⁴, X⁵ and X⁶ in above Formulas (IV) to (IX) include those illustrated and discussed above as representing Z¹ to Z¹¹, except condensated aryl radicals.

Illustrative radicals represented by Y¹, Y², Y³, Y⁴ and Y⁵ in above Formulas (IV) to (IX) include those illustrated and discussed above as representing Z¹ to Z¹¹, except condensated aryl radicals.

More preferably, X¹ is the same as X² and Z¹ is the same as Z² in Formula (IV), X³ is the same as X⁴, Z³ is the same as Z⁴, and Y¹, Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

Specific illustrative examples of the bisphosphite ligands employable in this invention include such preferred ligands as: 3,3'-bis(carboxyisopropyl)-1,1'-binaphthyl-2,2'-diyl-bis[bis(1-naphthyl)]phosphite having the formula: 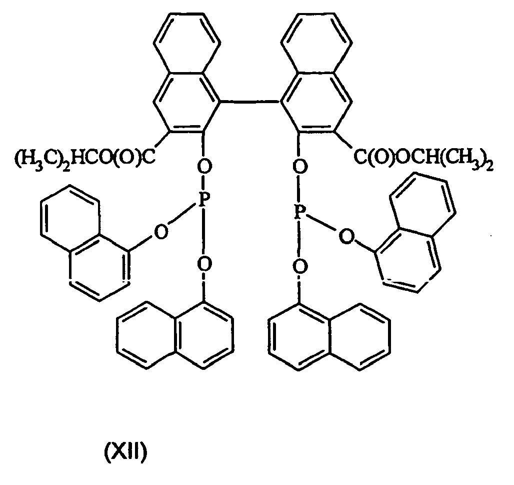 [3,3'-bis(t-butyl)-5,5'-dimethoxy-1,1'-biphenyl-2,2'-diyl]-bis(oxy)]-bis(dibenzo[d,f] [1,3,2])dioxaphosphepin having the formula: 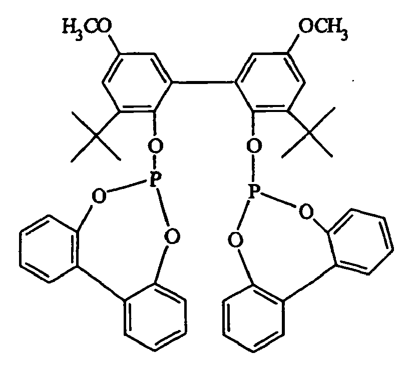 3,3'-bis(carboxyisopropyl)-1,1'-binaphthyl-2-yl-bis[(1-naphthyl)]phosphite-2'-yl-oxy-dibenzo[d,f] [1,3,2]dioxaphosphepin having the formula: 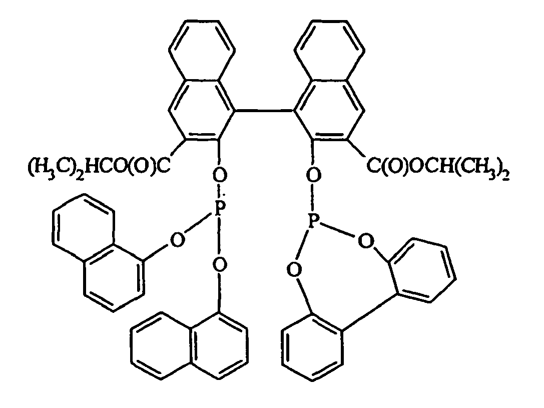 5,5'-bis(t-butyl)-3,3'-dimethoxy-1,1'-biphenyl-2,2'-diyl-bis[bis(1-naphthyl)]phosphite having the formula: 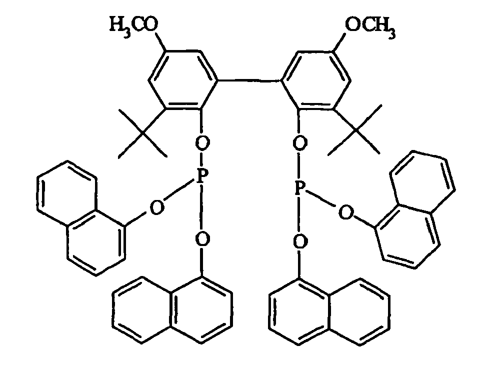 3,3'-bis(carboxymethyl)-1,1'-binaphthyl-2,2'-diyl-bis[bis(2-t-butylphenyl)]phosphite having the formula: 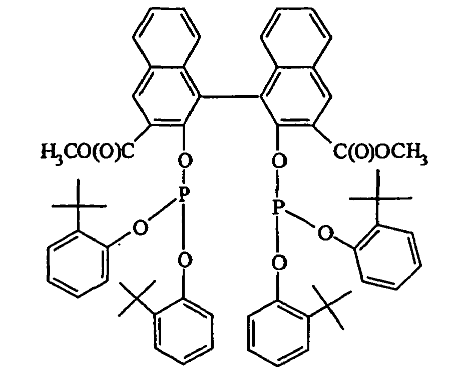 [3,3'-bis(t-butyl)-5,5'-dimethoxy-1,1'-biphenyl-2,2'-diyl]-bis(oxy)]-bis([1,1'-dinaphto[d,f] [1,3,2])dioxaphosphepin having the formula: 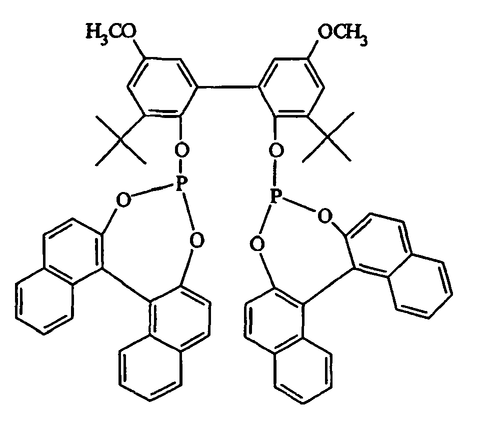 and the like.

Such types of bisphosphite ligands employable in this invention and/or methods for their preparation are well known as seen disclosed for example in U.S. Pat. Nos. 4,668,651; 5,288,918; 5,710,306, the entire disclosure of which is incorporated herein by reference thereto.

Of course it is to be understood that while the optimization of the subject invention necessary to achieve the best results and efficiency desired are dependent upon one's experience in the utilization of the subject invention, only a certain measure of experimentation should be necessary to ascertain those conditions which are optimum for a given situation and such should be well within the knowledge of one skilled in the art and easily obtainable by following the more preferred aspects of this invention as explained herein and/or by simple routine experimentation.

Finally, the aldehyde products of the hydroformylation process of this invention have a wide range of utility that is well known and documented in the prior art e.g. they are especially useful as starting materials for the production of alcohols and acids, as well as for the production of useful monomeric and polymeric compounds such as ε-caprolactam, nylon-6, adipic acid and nylon-6,6.

The following examples are illustrative of the present invention and are not be regarded as limitative. It is to be understood that all of the parts, percentages and proportions referred to herein and in the appended claims are by weight unless otherwise indicated.

### EXAMPLE 1

In a continuous process according to figure I the substrate methyl-3-pentenoate is reacted with CO and H2 to form a mixture of methyl formyl valerates. This is done by contacting methyl-3-pentenoate with a catalyst comprising Rh, 3,3'-bis(carboxyisopropyl)-1,1'-binaphthyl-2,2'-diyl-bis[bis(1-naphthyl)]phosphite ligand in slight molar excess to rhodium, and a mixture of CO and H2 (reaction conditions were as follows: 95°C, CO:H2 = 1:1 mole:mole, P = 0.5 MPa, mean residence time = 6 hours total in three well mixed reactors in series). The reactor effluent is flashed to 0.1 MPa to remove most of the dissolved gases which are subsequently purged. After cooling to less than 40 °C, the liquid is passed over a weakly basic ion exchange resin to remove acidic ligand degradation products. The catalyst concentration and free ligand concentration in the effluent is monitored regularly by on-line HPLC.

By feeding fresh ligand solution to the reactor section, a constant molar ratio of Rh:ligand of 1:1.2 mole:mole is maintained in the reactor effluent.

After flashing and passing the ion exchange resin, the reactor effluent is fed to a falling film evaporator operated at a pressure of 10 mbar. The evaporation rate is controlled by the temperature of the steam used as the heating utility for this evaporator. The steam temperature is controlled such that 75 wt% of the feed to the evaporator is evaporated which vapour stream is subsequently fed to a cyclone. The remaining 25 wt% contains almost all of the catalyst and is recycled to the reactor. The mean residence time of the catalyst in the separation unit is less than 10 minutes, of which less than 2 minutes are spent on the heating surface, the most critical area for the catalyst. 9% of the ligand (relative to the amount of ligand fed to the evaporator) degraded during the evaporation. The loss of rhodium in the product/catalyst separation is less than 0.015 % on weight.

At an operating pressure of 40 mbar and a steam temperature of 160°C, the ligand is almost quantatively degraded during the catalyst/product separation step. The following describe the lettered and numbered points in Figure I:
1. Ligand make-up stream
2. Other process feeds (e.g., CO, H₂, methyl-3-pentenoate)
3. Purge gases
4. Distillate to product work-up
5. Catalyst recycle stream
6. Heating oil
   A. Reactor section
   B. Flash section
   C. Ion exchange section
   D. Hot oil heated wiped film evaporator.

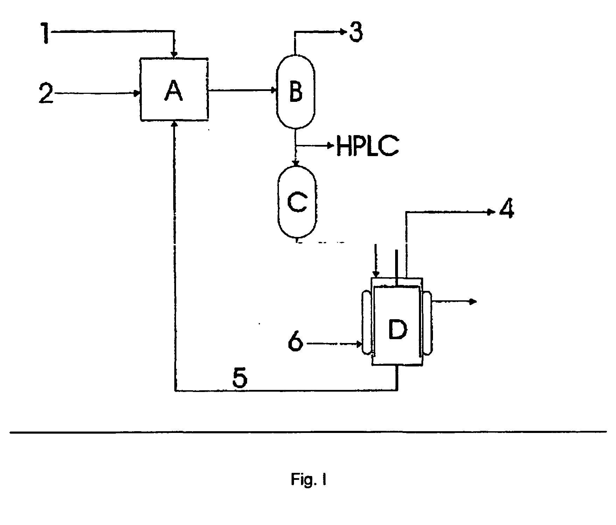

## Claims

1. A continuous hydroformylation process for forming an aldehyde comprising:
A) reacting an olefinically unsaturated compound containing from 6 to 30 carbon atoms, carbon monoxide, hydrogen, and a rhodium-bisphosphite ligand complex catalyst to form a reaction mixture,
B) separating aldehyde product from at least a portion of said reaction mixture, resulting in an aldehyde containing product stream and a catalyst containing product stream, and
C) recycling the catalyst containing stream to A),
wherein said separation is accomplished in a vacuum evaporator and a vapour/liquid separator and wherein the temperature of the heating surface of the evaporator is less than 125 °C and the residence time of the catalyst in the evaporator is less than 15 minutes.

2. The process of claim 2, wherein the heating surface of the evaporator is maintained at a temperature below 110°C.

3. The process according to claim 1 or 2, wherein the residence time is less than 10 minutes.

4. The process according to claim 3, wherein the residence time is less than 5 minutes.

5. The process of anyone of claim 1-4, wherein said evaporation is performed at a pressure below 50 mbar.

6. The process of claim 5, wherein said evaporation is performed at a pressure below 20 mbar.

7. The process of claim 6, wherein said evaporation is performed at a pressure below 10 mbar.

8. The process according to any one of claims 1-7, wherein the vacuum evaporator is a falling film evaporator.

9. Process according to anyone of claims 1-8, wherein the aldehyde is methyl-5-formylvalerate optionally in admixture with at least one of its isomers selected from methyl-4-formylvalerate, methyl-3-formylvalerate and methyl-2-formylvalerate.

10. The process of any one of daims 1-9, wherein the bisphosphite ligand of a formula selected from the group consisting of : 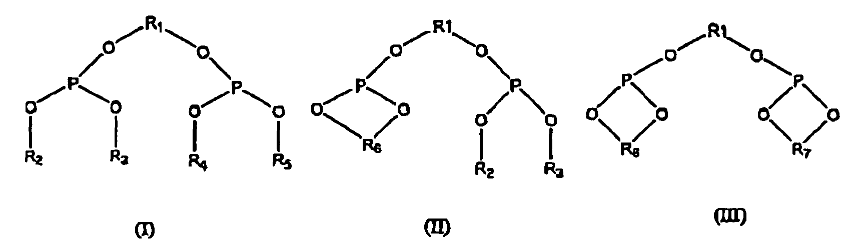 wherein each R¹ represents a divalent radical selected from a group consisting of alkylene, alkylene-(Q)ₙ,-alkylene, arylene and arylene-(Q)ₙ₋arylene, and wherein each alkylene radical individually contains from 2 to 18 carbon atoms and is the same or different, and wherein each arylene radical individually contains from 6 to 18 carbon atoms and is the same or different; wherein each Q individually represents a divalent bridging group of -O- or -CR'R"- wherein each R' and R" radical individually represents hydrogen or a methyl radical; and wherein each n individually has a value of 0 or 1.
wherein R², R³, R⁴, and R⁵ might be the same or different and each is individually represented by the structure of (VI) or (VII). 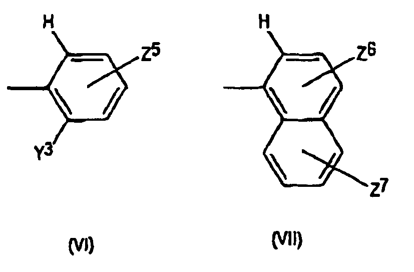 wherein R⁸ and R⁷ might be the same or different and each is individually represented by the structure of (VIII) or (IX), 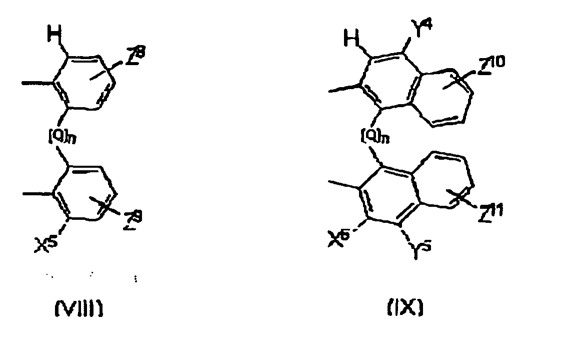 wherein X⁵ and X⁶ might be the same or different and each individually represents a hydrogen or an organic radical, wherein Y³, Y⁴ and Y⁵ are the same or different and each represents a hydrogen or alkyl radical,
wherein Z⁵, Z⁶, Z⁷, Z⁸, Z⁹, Z¹⁰ and Z¹¹ might be the same or different and each represent a hydrogen or an organic radical placed at any remaining position of the aryl rings.

11. The process of claim 10, wherein R¹ is represented by the structure of (IV). (V). 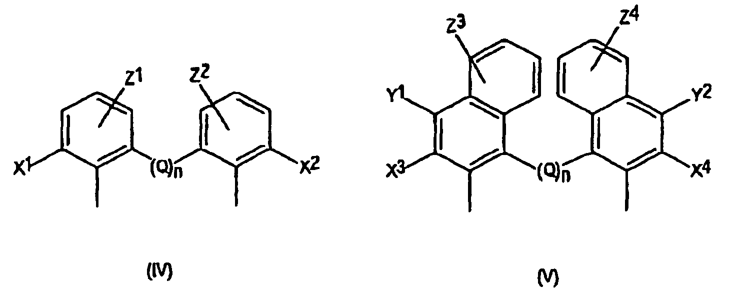 (VIII) or (IX), wherein X¹, X², X³, X⁴, X⁵ and X⁶ might be the same or different and each individually represents a hydrogen or an organic radical, wherein Y¹, Y², Y⁴ and Y⁵ are the same or different and each represents a hydrogen or an alkyl radical, wherein Z¹, Z², Z³, Z⁴, Z⁸, Z⁹, Z¹⁰ and Z¹¹ might be the same or different and each represent a hydrogen or an organic radical placed at any remaining position of the aryl rings of structures, wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁸ is the same as Z⁹ in Formula (VIII), Z¹⁰ is the same as Z¹¹ and Y⁴ and Y⁵ are hydrogen radicals in Formula (IX).

12. The process according to any one of claims 10-11, wherein the ligand used is [3,3'-bis(t-butyl)-5,5'-dimethoxy-1,1'-biphenyl-2,2'-diyl]-bis(oxy)]-bis(dibenzo[d,f][1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.

13. A continuous hydroformylation process for forming methyl-5-formylvalerate comprising:
A) reacting methyl-3-pentenoate, carbon monoxide, hydrogen, and a rhodium-bisphosphite ligand complex catalyst to form a reaction mixture comprising methyl-5-formylvalerate and catalyst.
B) separating methyl-5-formylvalerate from at least a portion of said reaction mixture, resulting in a methyl-5-formylvalerate containing product stream and a catalyst containing product stream, and
C) recycling the catalyst containing stream to A),
wherein said separation is accomplished in a vacuum falling film evaporator in combination with a vapour/liquid separator and wherein the temperature of the heating surface of the evaporator is less than 125°C and the residence time of the catalyst in the evaporator is less than 15 minutes.

14. Process according to claim 13, wherein the vapour liquid separator is a cyclone.

## Patentansprüche

1. Kontinuierliches Hydroformylierungsverfahren zum Bilden eines Aldehyds, umfassend:
A) das Umsetzen einer olefinisch ungesättigten Verbindung, die 6 bis 30 Kohlenstoffe enthält, Kohlenmonoxid, Wasserstoff und eines Rhodiumbisphosphitligandenkomplex-Katalysators unter Bildung eines Reaktionsgemisches,
B) das Abtrennen des Aldehydprodukts von mindestens einem Teil des Reaktionsgemisches, was in einem Aldehyd-enthaltenden Produktstrom und einem Katalysator-enthaltenden Produktstrom resultiert, und
C) das Rückführen des Katalysator-enthaltenden Stroms zu A),
wobei die Abtrennung in einem Vakuumverdampfer und einem Dampf/Flüssigkeit-Separator erreicht wird, und wobei die Temperatur der Heizfläche des Verdampfers weniger als 125°C beträgt, und die Verweilzeit des Katalysators in dem Verdampfer weniger als 15 Minuten beträgt.

2. Verfahren gemäß Anspruch 2, wobei die Heizfläche des Verdampfers bei einer Temperatur von unterhalb 110°C beibehalten wird.

3. Verfahren gemäß Anspruch 1 oder 2, wobei die Verweilzeit weniger als 10 Minuten beträgt.

4. Verfahren gemäß Anspruch 3, wobei die Verweilzeit weniger als 5 Minuten beträgt.

5. Verfahren gemäß einem der Ansprüche 1-4, wobei die Verdampfung bei einem Druck von unterhalb 50 mbar durchgeführt wird.

6. Verfahren gemäß Anspruch 5, wobei die Verdampfung bei einem Druck von unterhalb 20 mbar durchgeführt wird.

7. Verfahren gemäß Anspruch 6, wobei die Verdampfung bei einem Druck von unterhalb 10 mbar durchgeführt wird.

8. Verfahren gemäß einem der Ansprüche 1-7, wobei der Vakuumverdampfer ein Fallfilmverdampfer ist.

9. Verfahren gemäß einem der Ansprüche 1-8, wobei der Aldehyd Methyl-5-formylvalerat, gegebenenfalls im Gemisch mit mindestens einem von dessen Isomeren, ausgewählt aus Methyl-4-formylvalerat, Methyl-3-formylvalerat und Methyl-2-formylvalerat, ist.

10. Verfahren gemäß einem der Ansprüche 1-9, wobei der Bisphosphitligand von einer Formel ist, ausgewählt aus der Gruppe, bestehend aus: 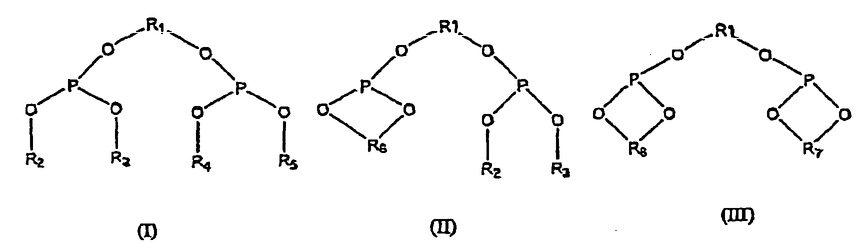 worin jedes R¹ einen zweiwertigen Rest darstellt, ausgewählt aus der Gruppe, bestehend aus Alkylen, Alkylen-(Q)ₙ-alkylen, Arylen und Arylen-(Q)ₙ-arylen, und wobei jeder Alkylenrest einzeln von 2 bis 18 Kohlenstoffatome enthält und gleich oder verschieden voneinander ist, und wobei jeder Arylenrest einzeln von 6 bis 18 Kohlenstoffatomen enthält und gleich oder voneinander verschieden ist, wobei jedes Q einzeln eine zweiwertige verbrückende Gruppe von -O- oder -CR'R"- ist, worin jeder Rest R' und R" einzeln Wasserstoff oder einen Methylrest darstellt, und wobei jedes n einzeln einen Wert von 0 oder 1 aufweist,
worin R², R³, R⁴ und R⁵ gleich oder verschieden voneinander sein können und jeweils einzeln durch die Struktur von (VI) oder (VII) dargestellt sind, 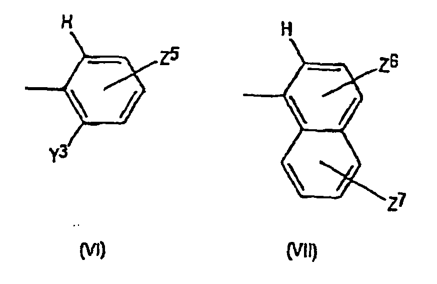 worin R⁶ und R⁷ gleich oder verschieden voneinander sein können und jeweils einzeln durch die Struktur von (VIII) oder (IX) dargestellt sind, 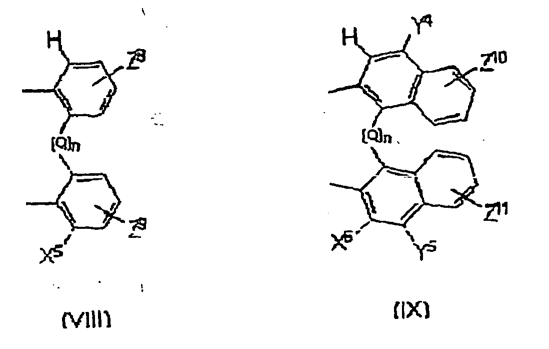 worin X⁵ und X⁶ gleich oder voneinander verschieden sein können und jeweils einen Wasserstoff oder einen organischen Rest darstellen, worin Y³, Y⁴ und Y⁵ gleich oder voneinander verschieden sind und jeweils ein Wasserstoffatom oder einen Alkylrest darstellen, worin Z⁵, Z⁶, Z⁷, Z⁸, Z⁹, Z¹⁰ und Z¹¹ gleich oder verschieden voneinander sein können und jeweils einen Wasserstoff oder einen organischen Rest, angeordnet an irgendeiner verbleibenden Stelle der Arylringe, darstellen.

11. Verfahren gemäß Anspruch 10, wobei R¹ durch die Struktur von (IV), (V) 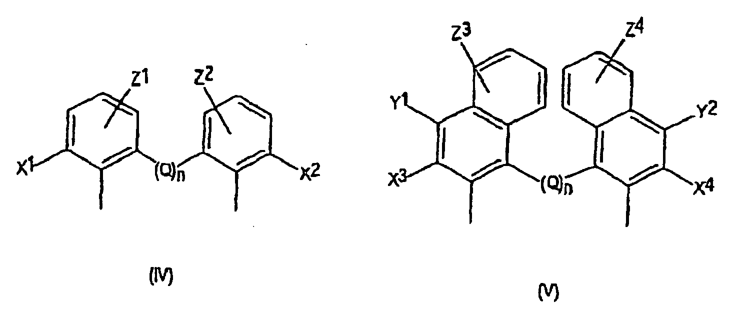 (VIII) oder (IX) dargestellt ist, worin X¹, X², X³, X⁴, X⁵ und X⁶ gleich oder verschieden voneinander sein können und jeweils einzeln ein Wasserstoffatom oder einen organischen Rest darstellen, worin Y¹, Y², Y⁴ und Y⁵ gleich oder verschieden voneinander sind und jeweils einen Wasserstoff oder einen Alkylrest darstellen, worin Z¹, Z², Z³, Z⁴, Z⁸, Z⁹, Z¹⁰ und Z¹¹ gleich oder verschieden voneinander sein können und jeweils ein Wasserstoffatom oder einen organischen Rest, angeordnet an irgendeiner vebleibenden Stelle der Arylringe der Strukturen, darstellen, worin X¹ der gleiche wie X² ist und Z¹ der gleiche wie Z² in Formel (IV) ist, X³ der gleiche wie X⁴ ist, Z³ der gleiche wie Z⁴ ist und Y¹ und Y² Wasserstoffreste in Formel (V) sind, Z⁸ der gleiche wie Z⁹ in der Formel (VIII) ist, Z¹⁰ der gleiche wie Z¹¹ ist und Y⁴ und Y⁵ Wasserstoffreste in Formel (IX) sind.

12. Verfahren gemäß einem der Ansprüche 10-11, wobei der verwendete Ligand [3,3'-Bis(t-butyl)-5,5'-dimethoxy-1,1'-biphenyl-2,2'-diyl]-bis(oxy)-bis(dibenzo[d,f])dioxaphosphepin, 3,3'-Bis(carboxyisopropyl)-1,1'-binaphthyl-2,2-diyl-bis[bis(1-naphthyl)]phosphit und 3,3'-Bis(carboxymethyl)-1,1'-binaphthyl-2,2'-diyl-bis[bis(2,5-di-t-butyl)]phosphit ist.

13. Kontinuierliches Hydroformylierungsverfahren zur Bildung von Methyl-5-Formylvalerat, umfassend:
A) das Umsetzen von Methyl-3-pentenoat, Kohlenstoffmonoxid, Wasserstoff und einem Rhodiumbisphosphitligandenkomplex-Katalysator, unter Bildung eines Reaktionsgemisches, umfassend Methyl-5-formylvalerat und Katalysator,
B) das Abtrennen von Methyl-5-formylvalerat von mindestens einem Teil des Reaktionsgemisches, was in einem Methyl-5-formylvalerat-enthaltenden Produktstrom und einem Katalysator-enthaltenden Produktstrom resultiert, und
C) das Rückführen des Katalysator-enthaltenden Stroms zu A),
wobei die Abtrennung in einem Vakuumfallfilmverdampfer in Kombination mit einem Dampf/Flüssigkeit-Separator erreicht wird, und wobei die Temperatur der Heizfläche des Verdampfers weniger als 125°C beträgt, und die Verweilzeit des Katalysators in dem Verdampfer weniger als 15 Minuten beträgt.

14. Verfahren gemäß Anspruch 13, wobei der Dampf/Flüssigkeit-Separator ein Cyclon ist.

## Revendications

1. Processus d'hydroformylation continu pour former un aldéhyde consistant à :
A) faire réagir un composé à insaturation oléfinique contenant de 6 à 30 atomes de carbone, du monoxyde de carbone, de l'hydrogène, et un catalyseur complexe de ligand rhodium-bisphosphite pour former un mélange réactionnel,
B) séparer le produit aldéhyde d'au moins une partie dudit mélange réactionnel, résultant dans un courant de produit contenant l'aldéhyde et un courant de produit contenant le catalyseur, et
C) recycler le courant contenant le catalyseur à A),
dans lequel ladite séparation est accomplie dans un évaporateur à vide et un séparateur de vapeur/liquide et dans lequel la température de la surface de chauffage est de moins de 125°C et le temps de résidence du catalyseur dans l'évaporateur est de moins de 15 minutes.

2. Processus selon la revendication 2, dans lequel la surface de chauffage de l'évaporateur est maintenue à une température au-dessous de 110°C.

3. Processus selon la revendication 1 ou 2, dans lequel le temps de résidence est de moins de 10 minutes.

4. Processus selon la revendication 3, dans lequel le temps de résidence est de moins de 5 minutes.

5. Processus selon l'une quelconque des revendications 1 à 4,
dans lequel ladite évaporation est réalisée à une pression au-dessous de 50 mbars.

6. Processus selon la revendication 5, dans lequel ladite évaporation est réalisée à une pression au-dessous de 20 mbars.

7. Processus selon la revendication 6, dans lequel ladite évaporation est réalisée à une pression au-dessous de 10 mbars.

8. Processus selon l'une quelconque des revendications 1 à 7, dans lequel l'évaporateur à vide est un évaporateur à couches minces.

9. Processus selon l'une quelconque des revendications 1 à 8, dans lequel l'aldéhyde est le méthyl-5-formylvalérate éventuellement mélangé à au moins un de ses isomères choisis parmi le méthyl-4-formylvalérate, le méthyl-3-formylvalérate et le méthyl-2-formylvalérate.

10. Processus selon l'une quelconque des revendications 1 à 9, dans lequel le ligand bisphosphite d'une formule choisie dans le groupe constitué par : 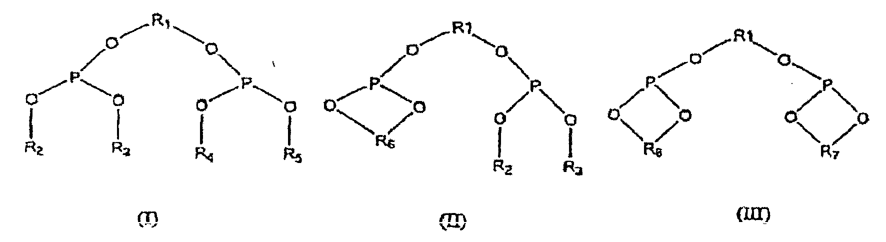 dans lesquelles chaque R¹ représente un radical divalent choisi dans un groupe constitué par un alkylène, un alkylène-(Q)ₙ₋alkylène, un arylène et un arylène-(Q)ₙ-arylène, et dans lequel chaque radical alkylène contient individuellement de 2 à 18 atomes de carbone et est identique ou différent, et dans lesquelles chaque radical arylène contient individuellement de 6 à 18 atomes de carbone et est identique ou différent ; dans lesquelles chaque Q représente individuellement un groupe de pontage divalent de -O- ou -CR'R"- dans laquelle chaque radical R' et R" représente individuellement un radical hydrogène ou méthyle ; et dans lesquelles chaque n a un individuellement une valeur de 0 ou 1,
dans lesquelles R², R³, R⁴ et R⁵ peuvent être identiques ou différents et chacun est représenté individuellement par la structure de (VI) ou (VII), 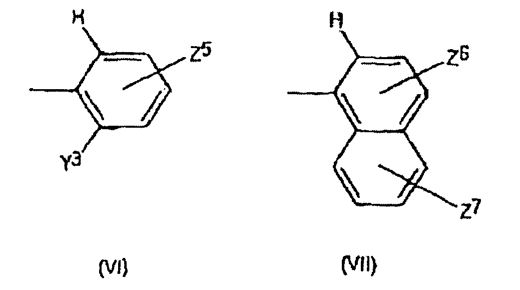 dans lesquelles R⁶ et R⁷ peuvent être identiques ou différents et chacun est représenté individuellement par la structure de (VIII) ou (IX), 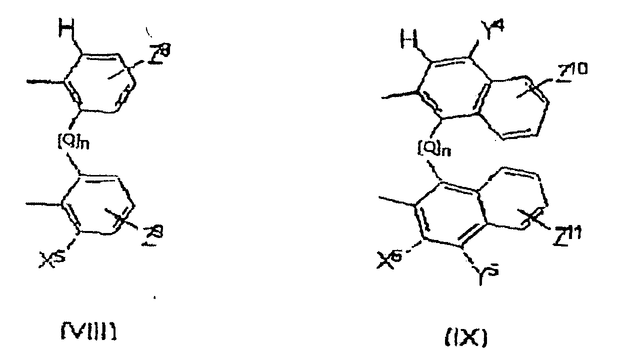 dans lesquelles X⁵ et X⁶ peuvent être identiques ou différents et chacun représente individuellement un hydrogène ou un radical organique, dans lesquelles Y³, Y⁴ et Y⁵ sont identiques ou différents et chacun représente un radical hydrogène ou alkyle, dans lesquelles Z⁵, Z⁶, Z⁷, Z⁸, Z⁹, Z¹⁰ et Z¹¹ peuvent être identiques ou différents et chacun représente un hydrogène ou un radical organique placé à n'importe quelle position restante des cycles aryle.

11. Processus selon la revendication 10, dans lequel R¹ est représenté par la structure de (IV), (V), 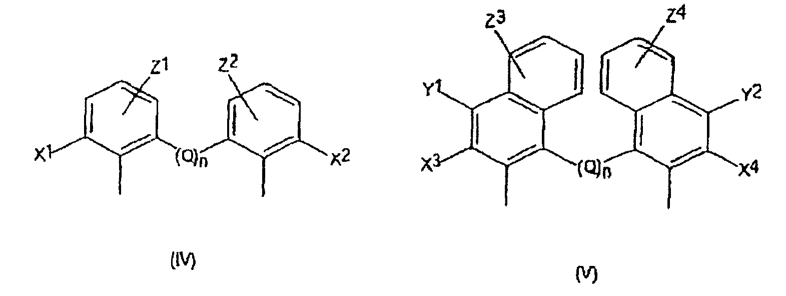 (VIII) ou (IX), dans lesquelles X¹, X², X³, X⁴, X⁵ et X⁶ peuvent être identiques ou différents et chacun représente individuellement un hydrogène ou un radical organique, dans lesquelles Y¹, Y², Y⁴ et Y⁵ sont identiques ou différents et chacun représente un hydrogène ou un radical alkyle, dans lesquelles Z¹, Z², Z³, Z⁶, Z⁸, Z⁹, Z¹⁰ et Z¹¹ peuvent être identiques ou différents et chacun représente un hydrogène ou un radical organique placé à toute position restante des cycles aryle des structures, dans lesquelles X¹ est identique à X² et Z¹ est identique à Z² dans la formule (IV), X³ est identique à X⁴, Z³ est identique à Z⁴, et Y¹ et Y² sont des radicaux hydrogène dans la formule (V), Z⁸ est identique à Z⁹ dans la formule (VIII), Z¹⁰ est identique à Z¹¹ et Y⁴ et Y⁵ sont des radicaux hydrogène dans la formule (IX).

12. Processus selon l'une quelconque des revendications 10 à 11, dans lequel le ligand utilisé est la [3,3'-bis(t-butyl)-5,5'-diméthoxy-1,1'-biphényl-2,2'-dlyl]-bis(oxy)]-bis(dibenzo-[d,f]-[1,3,2]dioxaphosphépine, le 3,3'-bis(carboxylisopropyl)-1,1'-binaphtyl-2,2dlyl-bis[bis(1-naphtyl)phosphite et le 3,3'-bis-(carboxyméthyl)-1,1'-binaphtyl-2,2'-dlyl-bis[bis(2,5-di-t-butyl)]-phosphite.

13. Processus d'hydroformylation continu pour former le méthyl-5-formylvalérate consistant à :
A) faire réagir du méthyl-3-penténoate, du monoxyde de carbone, de l'hydrogène et un catalyseur complexe de ligand rhodium-bisphosphite pour former un mélange réactionnel comprenant le méthyl-5-formylvalérate et le catalyseur,
B) séparer le méthyl-5-formylvalérate d'au moins une partie du mélange réactionnel, résultant dans un courant de produit contenant le méthyl-5-fomylvalérate et un courant de produit contenant le catalyseur, et
C) recycler le courant contenant le catalyseur à A),
dans lequel ladite séparation est accomplie dans un évaporateur à couches minces à vide en combinaison avec un séparateur de vapeur/liquide et dans lequel la température de la surface de chauffage de l'évaporateur est de moins de 125°C et le temps de résidence du catalyseur dans l'évaporateur est de moins de 15 minutes.

14. Processus selon la revendication 13, dans lequel le séparateur de vapeur liquide est un cyclone.
